# EUROPEAN PATENT SPECIFICATION

(11) **EP 3 917 475 B1**
(45) Date of publication and mention of the grant of the patent: **04.06.2025**
(21) Application number: 20749049.1
(22) Date of filing: 27.01.2020
(51) Int. Cl.: A61F 13/00, A61F 13/02, A61M 1/00, A61L 15/18, A61L 15/28, A61L 15/42, A61L 15/44, A61L 15/60, A61L 15/64, A61L 24/00

(54) **WOUND DRESSING COMPOSITIONS AND METHODS**
WUNDVERBANDZUSAMMENSETZUNGEN UND VERFAHREN
COMPOSITIONS DE PANSEMENT ET PROCÉDÉS

(30) Priority: 28.01.2019 US 201962797576 P
(43) Date of publication of application: 08.12.2021
(73) Proprietor: Core Scientific Creations Ltd., 4926918 Petah Tiqva (IL)
(72) Inventor: ELIYAHU-GROSS, Shani, 4926918 Petah Tiqva (IL); YASKIL, Yuval, 4926918 Petah Tiqva (IL)
(74) Representative: Murgitroyd & Company
(86) International application number: PCT/IB2020/050617
(87) International publication number: WO 2020/157633

(56) References cited:
- US-A1- 2014 234 419
- US-A1- 2015 283 287
- US-A1- 2016 121 019
- US-A1- 2018 028 713
- US-B2- 7 060 056
- US-B2- 7 744 644
- US-B2- 7 854 923

## Description

### Cross-Reference to Related Applications

This application claims priority to and the benefit of U.S. Provisional Patent Application No. 62/797,576 filed January 28, 2019.

### Background

**.**

Hemostats can be grossly divided into two types of bleeding control products: bioabsorbable and non-dissolvable hemostats. The vast majority of dissolvable hemostats are based on an oxidized cellulosic structure (e.g., oxidized (regenerated) cellulose). While bioabsorbable, cellulosic hemostats composed of non-oxidized carboxymethyl-cellulose (CMC) have been described for non-compressional use for bleeding control, control of specific end product properties has been lacking. See, e.g., WO 2016/067266 A1; WO 2016/067250 A1; US 2017/0335017 A1; and US 2016/0121019 A1. What is needed is a combination hemostatic product and methods to control the end product properties by combining two or more components together.

### Summary

The invention is defined in the independent claims 1 and 8. Aspects described herein provide hybrid wound dressings (e.g., hemostat) comprising bio-absorbable polyelectrolyte material embedded with ionic crystals (e.g., salt) and its clinical advantages.

In yet another aspect, hybrid wound dressings comprising bio-absorbable polyelectrolyte material embedded with ionic crystals (e.g., salt) which are utilized to control and change the polymeric component properties in general, and specifically under the clinical environment of bleeding wounds, are provided. The controllable properties are, for example, the hydrophilic/hydrophobic nature of the product, its interaction with the body, adherence, percent absorbance, biodegradation rate, clotting strength, charge, and acidity.

Polyelectrolytes are polymers whose repeating units bear an electrolyte (ionic) group. These groups dissociate in aqueous solutions, making the polymers charged.

In another aspect, the wound dressings comprise non-oxidized CMC/NaCl gauze product. Further aspects described herein characterize the degree of substitution (DS) and salt levels within the hybrid hemostat, as a way to control components ratio and influence on the overall behavior of the product, and its applications.

### Brief Description of the Drawings

Figure 1 provides an exemplary chemical reaction to produce CMC/NaCl product and;
Figure 2 illustrates an exemplary water absorption profile, which was conducted over 24 hours before and after (de-salted) sonication in various solutions: (1) deionized water (DI), (2) 0.9% NaCl saline, and (3) 2% NaCl saline.

### Detailed Description

### I. Salt embedded WoundClot

Aspects described herein provide exemplary manufacturing process utilizing saturated solution (described in IV below). In this aspect, the high constant concentration and the presence of, for example, inorganic salt sedimentation during manufacturing results in the production of salt-embedded hemostat.

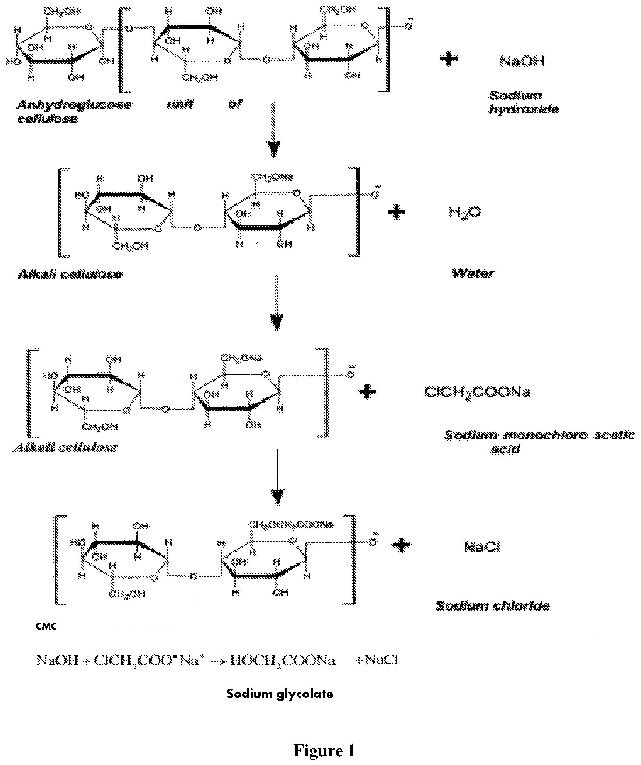

Further aspects describe methods of making wound dressings using, for example, three different salts: sodium mono-chloroacetic acid (ClCH₂COONa) (CAANa) reactant, sodium glycolate (OHCH₂COONa), and sodium chloride (NaCl), as byproducts (Figure 1). [Reference 1]. Since the reaction occurs in an organic solvent, the NaCl inorganic salt retains its solid form in crystal shape with low solubility. The inorganic salt crystals are dispersed in the reaction medium and are trapped by the treated fabric's porous structure, while the organic salts are washed away with the organic medium.

Alternatively, one can produce CMC material according to common procedures (non-saturated solution with lower organic salt CAANa formation) and intentionally add the required amount of salt onto the CMC product to embed the salt in the wound dressings and provide specific properties as described herein.

In one aspect, the salt's chemical structure is monovalent. Without being bound by theory, it is believed that higher electrovalent salts may result in crosslinking and elimination of the bio-absorbability properties of the wound dressing.

HPLC-LCMS analysis was used to determine residual organic salts in the form of CAA derivatives in an exemplary hybrid hemostat. The hybrid hemostat products were found to contain, for example, residual 0.29 wt% CAA derivatives, on average. It is believed that CAA derivatives have no significant effect in this concentration on the exemplary hybrid hemostat.

### II. Salt for controlling hemostatic properties

Aspects provided herein utilize different salt amounts to control the behavior properties of the exemplary bio-absorbable hybrid hemostat.

Without being bound by theory, it is believed that the basic hemostat-body interaction is dominated by the polyelectrolyte-aqueous liquid physical interactions nature. It is a well-known physical principle that the more polar the molecule, the faster it will dissolve in an aqueous surrounding. The polar nature of polyelectrolytes is affected, for example, by:
A) the amount of charges along the polymeric chain
B) the ionic dissociation ratio (ID) of those charges.

With respect to CMC, the ionic groups can be determined by measuring the degree of substitution (DS) (e.g., by titration or Fourier-transform infrared spectroscopy (FTIR)) as every ionic group is added to the cellulose structure. The relevant range of DS of the present CMC hemostat is between, for example, 0.6-1.4 and most commonly 0.7-1.2.

Aspects described herein can be directed to controlling the hemostat-body interaction based on the ionic dissociation ratio.

Several theories can explain the charge-solution interactions that result in the properties described herein. For example, the Poisson-Boltzmann equation can take many forms throughout various scientific fields. In biophysics and certain surface chemistry applications, it is known simply as the Poisson-Boltzmann equation. It is also known in electrochemistry as Gouy-Chapman theory; in solution chemistry as Debye-Huckel theory; in colloid chemistry as Derjaguin-Landau-Verwey-Overbeek (DLVO) theory. All of these theories relate to the interaction of existing charge and the way its interaction with the environment is affected by the presence of other charges. Generally speaking, a higher concentration of present ions results in screening of specific charge and reducing its potential interaction in the environment.

In one aspect, the embedded solid salt crystals within an exemplary dry CMC hemostat have an important role once the product is exposed to liquid (blood/exudates). As the product starts to absorb the liquid, the monovalent crystals locally dissolve around the area of contact with the liquid and release free ions of the polyelectrolyte to the local surrounding area. The more salt embedded on the CMC product results in higher the local concentration of free ions upon exposure to liquid. Higher free ionic concentration affects the dissociation ratio of the CMC's bound ionic groups, which are reduced in number, and the polymeric chain becomes less charged.

Without being bound by theory, it is believed that the absorption rate of the polymer is reduced, and related properties are modified. This effect can cause a temporary change in the behavior of the CMC hemostat due to the dynamic behavior of the overall system. Once the chemical potential is evenly distributed across the polymeric chain, the system returns to its normal behavior. The advantage of the temporary effect is that it can occur and then resolve back to normal behavior in the time frame of treating a bleeding wound and staunching blood flow. By increasing the salt amount, one can reinforce the gel formation, which aids effective bleeding control by slowing or stopping blood flow from the wound through blockage of the flow with the gelatinous form of the product, and slow the rate of dissolution for the gelatinous form of product. Hence, severe bleeding can be treated with ease, while preserving the bio-absorbable property of the CMC product for later in the treatment process.

Aspects described herein provide a wound dressing comprising an absorbable polyelectrolyte material and ionic crystals, wherein the weight percent of ionic crystals in the absorbable polyelectrolyte material is at least about 5% w/w of the overall weight.

The term "wound dressing" refers to a device, material, or substance adapted to be applied to or associated with an internal or external body wound or injury to aid in treatment or amelioration of a wound or injury, and is typically sterilized prior to use. A wound dressing can include a supporting structure and other active or inactive ingredients to aid in treatment or amelioration of the wound or injury.

The term "polyelectrolyte" refers to a polymer with repeating units bearing an electrolyte group.

In another aspect, ionic crystals (e.g., NaCl, NaBr, Nal, NaF, KCI, KBr, KI, and KF) are embedded in the absorbable polyelectrolyte material. In a further aspect, the weight percent of ionic crystals is about 20 to 70% w/w of the overall weight. The ionic crystals can be, for example, divalent or trivalent salt crystals.

The polyelectrolyte material can be bioabsorbable (e.g., non-oxidized carboxymethyl cellulose). In this aspect, the degree of substitution of the non-oxidized carboxymethyl cellulose can be from about 0.3-1.8 or from about 0.75-1.2.

In this aspect, the degree of polymerization of the non-oxidized carboxymethyl cellulose can be from about 20-3500 or from about 100-1500.

In another aspect, the polyelectrolyte material is selected from the group consisting of bioresorbable, biodegradable, and bioabsorbable polyelectrolyte material. In yet another aspect, the polyelectrolyte material is selected from the group consisting of non-oxidized polysaccharide, oxidized polysaccharide, regenerated polysaccharide, non-regenerated polysaccharide, bleached polysaccharide, unbleached polysaccharide, mercerized polysaccharide, un-mercerized polysaccharide, scoured polysaccharide, natural polysaccharide, or combinations thereof.

In a further aspect, the polyelectrolyte material comprises a polyelectrolyte ionic group selected from the group consisting of monovalent cations, monovalent anions, divalent cations, divalent anions, trivalent cations, and trivalent anions. In another aspect, the polyelectrolyte material can comprise one or more polyelectrolyte ionic groups selected from the group consisting of monovalent cations, monovalent anions, divalent cations, divalent anions, trivalent cations, and trivalent anions

In yet another aspect, the ionic crystals comprise an ionic group selected from the group consisting of monovalent cations, monovalent anions, divalent cations, divalent anions, trivalent cations, and trivalent anions.

In one aspect, the valence of the polyelectrolyte ionic group and the ionic crystals are the same. In another aspect, the valence is selected from the group consisting of 1, 2, and 3.

The wound dressing can be formed from a material selected from the group consisting of a powder, a film, a fabric, a foam, yarns, fibers, a coating, a solution, a gel, or a combination thereof. The wound dressing can be in the form of a hemostat or bandage. In another aspect, the fabric can be selected from the group consisting of woven, non-woven, and knitted fabrics.

In yet another aspect, the wound dressing further comprises an active ingredient (e.g., an antibiotic, an antimicrobial, analgesic, a clotting agent, a steroid, a wound healing agent, a cooling agent, and an electric potential agent). The antibiotic can be selected from the group consisting of cefazolin, erythromycin, and cefoxitin. The antimicrobial can be selected from the group consisting of antiseptics and anti-microbial peptides. The analgesic can be selected from the group consisting of acetaminophen, ibuprofen, naproxen, celecoxib, rofecoxib, etoricoxib, codeine, oxycodone, hydrocodone, dihydromorphine, pethidine, tramadol, buprenorphine, alcohol, and cannabis. The steroid can be selected from the group consisting of androgens, anabolic steroids, antiandrogens, estrogens, progestogens, corticosteroids, and neurosteroids. The wound healing agent can be selected from the group consisting of silver sulphadiazene, silver nitrate, povidone-iodine, chlorohexidine, and polyhexamethylene biguanide.

In another aspect, the absorption percent of the wound dressing is from about 2000 to about 4000 percent over about 24 hours.

### III. Methods of Manufacturing Wound Dressings

Aspects described herein provide methods of making a wound dressing, comprising treating cellulose with an alkali solution; mixing the alkali solution with a chloracetic acid (CAA) solution to form a saturated sodium chloroacetate (NaCAA) solution, wherein ionic crystals are formed in the NaCAA solution; and treating the cellulose with the NaCAA solution to form carboxy methyl cellulose (CMC) having embedded ionic crystals, wherein the weight percent of ionic crystals in the absorbable polyelectrolyte material is at least about 5% w/w of the overall weight. In this aspect, CMC can be washed with ethanol. In another aspect, the CMC can be neutralized with an acid.

In another aspect, the weight percent of ionic crystals is about 20 to 70% w/w of the overall weight.

In a further aspect, the polyelectrolyte material is non-oxidized carboxymethyl cellulose. In yet another aspect, the degree of substitution of the non-oxidized carboxymethyl cellulose is from about 0.3-1.8. The degree of substitution of the non-oxidized carboxymethyl cellulose can be from about 0.75-1.2. In a further aspect, degree of polymerization of the non-oxidized carboxymethyl cellulose is from about 20-3500 or 100-1500.

In another aspect, the alkali solution comprises sodium hydroxide and a solvent. The solvent can be selected from the group consisting of H₂O and ethanol. In another aspect, the CAA solution can comprise CAA and a solvent. The solvent can be selected from the group consisting of H₂O, ethanol, others.

In one aspect, mixing the alkali solution with a chloracetic acid (CAA) solution is performed at around 50 degrees C.

In another aspect, the cellulose is treated with the NaCAA solution at about 50 to about 60 degrees C for about 12 to 14 hours.

Methods of making a wound dressing, comprising associating an absorbable polyelectrolyte material with ionic crystals, wherein the weight percent of ionic crystals associated with the absorbable polyelectrolyte material is at least about 5% w/w of the overall weight are provided.

In this aspect, the ionic salt crystals are substantially associated with the absorbable polyelectrolyte material. The term "substantially associated" means at least about 50%, 60%, 70%, 80%, or 90% of the salt crystals are in proximity of or connected physically or chemically with the absorbable polyelectrolyte material (e.g., by mechanical force, coating, impregnating, deposition, or embedded in).

In another aspect, the weight percent of the ionic crystals is about 20 to 70% w/w of the overall weight. In a further aspect, the ionic crystals are selected from the group consisting of NaCl, NaBr, Nal, NaF, KCI, KBr, KI, and KF. The ionic crystals can be selected from the divalent or trivalent salt crystals.

In another aspect, the polyelectrolyte material is non-oxidized carboxymethyl cellulose. The degree of substitution of the non-oxidized carboxymethyl cellulose can be from about 0.3-1.8 or 0.75-1.2.

In another aspect, the degree of polymerization of the non-oxidized carboxymethyl cellulose is from about 20-3500 or 100-1500.

The polyelectrolyte material can be selected from the group consisting of bioresorbable, biodegradable, and bioabsorbable. The polyelectrolyte material can be selected from the group consisting of non-oxidized polysaccharide, oxidized polysaccharide, regenerated polysaccharide, non-regenerated polysaccharide, bleached polysaccharide, unbleached polysaccharide, mercerized polysaccharide, un-mercerized polysaccharide, scoured polysaccharide, natural polysaccharide, or combinations thereof.

The polyelectrolyte material can comprise a polyelectrolyte ionic group selected from the group consisting of monovalent cations, monovalent anions, divalent cations, divalent anions, trivalent cations, and trivalent anions.

In another aspect, the ionic crystals can comprise an ionic group selected from the group consisting of monovalent cations, monovalent anions, divalent cations, divalent anions, trivalent cations, and trivalent anions.

The valence of the polyelectrolyte ionic group and ionic crystals can be the same. The valence can be selected from the group consisting of 1, 2, and 3.

In another aspect, the wound dressing can be formed from a material selected from the group consisting of a powder, a film, a foam, a fabric, yarns, fibers, a coating, a solution, a gel, or a combination thereof.

The wound dressing can be in the form of a hemostat or bandage. The fabric can be selected from the group consisting of woven, non-woven, and knitted fabrics.

In this aspect, the wound dressing can further comprise an active ingredient (e.g., an antibiotic, an antimicrobial, analgesic, a clotting agent, a steroid, a wound healing agent, a cooling agent, and an electric potential agent).

Aspects described herein provide methods of increasing the water affinity of the wound dressings described herein by decreasing the weight percent of ionic crystals in the absorbable polyelectrolyte material down to approximately 5% w/w of the overall weight.

Further aspects provide methods of increasing the absorption of body fluids by the wound dressings described herein by decreasing the weight percent of ionic crystals in the absorbable polyelectrolyte material down to approximately 5% w/w of the overall weight.

Yet further aspects provide methods of increasing the gel stability of the wound dressings described herein by increasing the weight percent of ionic crystals in the absorbable polyelectrolyte material up to approximately 90% w/w of the overall weight.

Further aspects provide methods of increasing the topographic adjustment potential of the wound dressings described herein by decreasing the weight percent of ionic crystals in the absorbable polyelectrolyte material down to approximately 5% w/w of the overall weight.

Aspects described herein provide methods of increasing the adhesion of the wound dressings described herein to a wound by decreasing the weight percent of ionic crystals in the absorbable polyelectrolyte material down to approximately 5% w/w of the overall weight.

Aspects described herein provide methods of increasing the clot activation time for a wound covered by the wound dressings described herein by increasing the weight percent of ionic crystals in the absorbable polyelectrolyte material up to approximately 90% w/w of the overall weight.

Aspects described herein provide methods of decreasing the swelling associated with a wound covered by the wound dressings described herein by decreasing the weight percent of ionic crystals in the absorbable polyelectrolyte material down to approximately 5% w/w of the overall weight.

### IV. Results and Discussion

Exemplary lab experiments were conducted to assess the influence of salt concentration at the local surrounding area near the polyelectrolyte material.

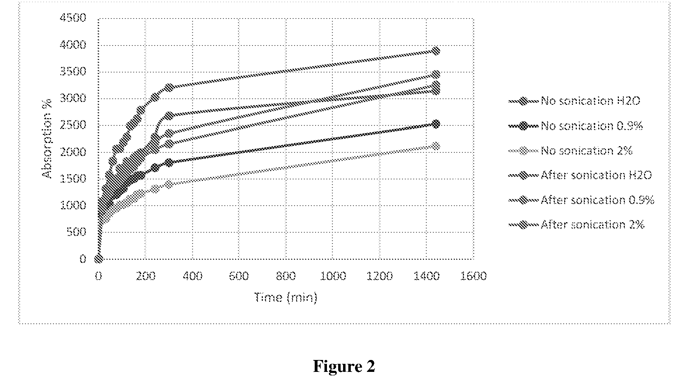

Figure 2 shows the influence of NaCl concentration on water adsorption by the product, where higher salt concentration reduces the sample's adsorption ability. The influence of the salt was evaluated by two methods-(1) measuring the amount of salt in the embedded product (reduced by sonication treatment) and (2) measuring the median salt concentration exposed to the product.

According to Figure 2, non-sonicated samples, which had higher salt concentrations, gave a lower water absorption profile. The decrease in water absorption was comparable to samples that were absorbed from saline solutions, containing 0.9 wt% and 2 wt% NaCl. These results show that higher salt concentrations in the sample or in the absorption solution reduces the product's water adsorption ability.

Free chlorides tests that were performed on dissolved samples, before and after sonication, showed that the CMC/NaCl product is composed of two forms of NaCl crystals within the gauze: a covering layer of 5-15% wt, which can be washed away by sonication, and an internal embedded layer of 5-50% wt, which can be dissolved during the gauze dissolution. The NaCl content in the product influences and controls the product's properties upon application for wound healing purposes and applications.

When a polyelectrolyte chain is immersed in aqueous solution of electrolyte, the electrostatic field around the chain increases the local concentration of the diffuse counter-ions and decreases one of the co-ions. The difference between the two concentrations is maximal at the chain surface, and diminishes quasi-exponentially down to zero in the bulk.

The distribution of the local concentration of the free ions can be determined by the ratio between the electrostatic and the thermal energies. In this aspect, when this ratio increases above a critical value, part of counter-ions are electrostatically adsorbed on the chain, forming temporary ion pairs with the electric charges of the polymer. This phenomenon is known as counter-ion condensation. [Reference 2]

This behavior typically demonstrates the Debye-Hückel theory of non-ideality of electrolyte solutions. [Reference 3]. According to Debye-Hückel theory, non-ideality arises principally from ions of opposite charges that attract each other, while ions of the same charge repel each other, due to electrostatic forces. As a result, an electrical double layer (EDL) is created near the sample surfaces, and has a significant influence on its water absorbance capacity: higher ionic (salt) concentrations in the absorbed solution, will mask the CMC hydrophilic properties, and reduce the water absorbance and the solubility rate of the product, and will also be expressed in a stronger gel.

In this example, the concentration of the chloride ions (Cl⁻) has the most influence on the clot structure. Under physiological conditions, the presence of Cl⁻ can promote aggregation, by influencing the fibrin fiber thickness during the coagulation process. Chlorides alter the rate of dissociation of certain side chains of the protein chains, and alter their tendency to react and clump together. Hence, manipulating the salt around a wound can significantly alter the structure, thickness and strength of the clot. [Reference 4].

Controlling the NaCl in the product can modify the product for a specific application target. For example, higher NaCl leveled products can retain lower absorbance rates and prolong bio-degradation process. This property could be applied to severe bleeding injuries where stiffer and more stable products are required. The gradual dissociation of the ions provides prolonged activation time of Hageman factors, and promotes the natural clotting cascade. Lower NaCl leveled products can be used for high absorption rate with short term stability.

### V. Exemplary Manufacturing process for salt embedded CMC

Tank 1: Reactor vessel
Tank 2: Mixing vessel (for solution preparation)

Fabric gauze is rolled and placed in tank 1. Fabric gauze can also be placed by any suitable method (e.g., folded, staged). The gauze is the cellulose raw material to be modified by the chemical reaction. The same reaction can also be used for other carbohydrates substrates (e.g., starch or chitin) that can undergo etherification reaction to form the carboxy-methyl substitution group onto the substrate.

In tank 2 the alkali solution (NaOH _{(aq)}) is mixed in ethanol solvent for 30 minutes.

The content of tank 2 is transferred to tank 1 with the fabric, mixed for 4 hours to form alkali cellulose (i.e., the activation stage to open the cellulose crystals to the reaction stage.

Mixing chloroacetic acid (CAA) with water and ethanol in the mixing vessel (tank 2) to create CAA homogenous solution.

The medium from tank 1 (the mixture of the solvent with the alkali solution) is transferred gradually to tank 2 under stirring conditions. The mixing between CAA and the Alkali substance (NaOH) results in the transformation of the CAA to its salt state, sodium chloro acetate-NaCAA. Since NaCAA has limited solubility in the medium (i.e., the mixture of ethanol with water from the alkali solution) the NaCAA dissolves to the maximum capacity, the medium become saturated and the rest of the formed salt of NaCAA is in solid state as sediment in the medium. This stage is exothermic and maintained at 50°C.

Once the saturated solution of NaCAA is formed in tank 2, all the substance (Medium + sedimentation) is transferred to tank 1-to the alkali cellulose. In this reaction (etherification) stage, the NaCAA molecule is chemically bound to the cellulose substrate and forms carboxy methyl cellulose (CMC). For each substitution (i.e., the bounding of one NaCAA molecule to the cellulose), one molecule of NaCl is formed. In this aspect, the solubility of NaCl in the medium is very limited (e.g., solubility of NaCl in ethanol is 0.65/kg) so the salt formed as solid crystals which are trapped between the fabric fibers. The reaction can be maintained at 50-60°C for 12-14 hours.

Upon reaction, the medium is washed away with ethanol to remove alkali residues and neutralized with HCl solution at ambient temperature for 12 hours. In this aspect, the neutralization reaction can be NaOH + HCl → NaCl + H₂O so that additional NaCl is provided during neutralization of the CMC.

Once the fabric is neutralized, the ethanol is transferred to waste, and the fabric transferred to a drying stage outside of the reactor (e.g., evaporating the ethanol with air).

Then the product is cut, fold, packed, and sterilized.

While the aspects described herein have been disclosed with reference to certain embodiments, numerous modifications, alterations, and changes to the described embodiments are possible without departing from the sphere and scope of the present invention, as defined in the appended claims. Accordingly, it is intended that the present invention not be limited to the described embodiments, but that it has the full scope defined by the language of the following claims, and equivalents thereof.

### References

1. Saputra et al., Int. J. Chem. Eng. Appl., 2014, 5(1), 36-40.
2. A-M. Zhivkov (2013). Electric Properties of Carboxymethyl Cellulose, Cellulose - Fundamental Aspects, Dr. Theo G.M. Van De Ven (Ed.).
3. P. Debye and E. Hückel, Physikalische Zeitschrift. 1923, 24, 185-206.
4. Di Stasio et al., Biophysical J. 1998, 75, 1973-1979.

## Claims

1. A wound dressing comprising an absorbable polyelectrolyte material and ionic crystals, wherein a weight percent of ionic crystals in the absorbable polyelectrolyte material is at least about 5% w/w of an overall weight.

2. The wound dressing of claim 1, wherein the ionic crystals are embedded in the absorbable polyelectrolyte material.

3. The wound dressing of claim 1, wherein the weight percent of ionic crystals is about 20 to 70% w/w of the overall weight.

4. The wound dressing of claim 1, wherein the polyelectrolyte material is selected from the group consisting of bioresorbable, biodegradable, and bioabsorbable.

5. The wound dressing of claim 1, wherein the wound dressing is formed from a material selected from the group consisting of a powder, a film, a fabric, a foam, yarns, fibers, a coating, a solution, a gel or a combination thereof.

6. The wound dressing of claim1, wherein the wound dressing is in the form of a hemostat or bandage.

7. The wound dressing of claim 1, further comprising an active ingredient, wherein the active ingredient is selected from group consisting of an antibiotic, an antimicrobial, an analgesic, a clotting agent, a steroid, a wound healing agent, a cooling agent, and an electric potential agent.

8. A method of making a wound dressing as defined in any of claims 1 to 7, comprising:
treating cellulose with an alkali solution;
mixing the alkali solution with a chloracetic acid (CAA) solution to form a saturated sodium chloroacetate (NaCAA) solution, wherein ionic crystals are formed in the NaCAA solution; and
treating the cellulose with the NaCAA solution to form carboxy methyl cellulose (CMC) having embedded ionic crystals, wherein a weight percent of ionic crystals in the absorbable polyelectrolyte material is at least about 5% w/w of an overall weight.

9. A method of making a wound dressing as defined in any of claims 1 to 7, comprising associating an absorbable polyelectrolyte material with ionic crystals, wherein a weight percent of ionic crystals associated with the absorbable polyelectrolyte material is at least about 5% w/w of an overall weight.

10. The method of claim 9, wherein the ionic salt crystals are substantially associated with the absorbable polyelectrolyte material.

11. The method of claim 9, wherein the weight percent of ionic crystals is about 20 to 70% w/w of the overall weight.

12. The method of claim 9, wherein the polyelectrolyte material is selected from the group consisting of bioresorbable, biodegradable, and bioabsorbable.

13. The method of claim 9, wherein the wound dressing is formed from a material selected from the group consisting of a powder, a film, a foam, a fabric, yarns, fibers, a coating, a solution, a gel or a combination thereof.

14. The method of claim 9, wherein the wound dressing is in the form of a hemostat or bandage.

15. The method of claim 9, wherein the wound dressing further comprises an active ingredient, wherein the active ingredient is selected from group consisting of an antibiotic, an antimicrobial, analgesic, a clotting agent, a steroid, a wound healing agent, a cooling agent, and an electric potential agent.

## Patentansprüche

1. Ein Wundverband, der ein absorbierbares Polyelektrolytmaterial und lonenkristalle beinhaltet, wobei ein Gewichtsprozent von lonenkristallen in dem absorbierbaren Polyelektrolytmaterial mindestens etwa 5 Gew.-% eines Gesamtgewichts beträgt.

2. Wundverband gemäß Anspruch 1, wobei die lonenkristalle in dem absorbierbaren Polyelektrolytmaterial eingebettet sind.

3. Wundverband gemäß Anspruch 1, wobei das Gewichtsprozent von lonenkristallen etwa 20 bis 70 Gew.-% des Gesamtgewichts beträgt.

4. Wundverband gemäß Anspruch 1, wobei das Polyelektrolytmaterial aus der Gruppe ausgewählt ist, die aus bioresorbierbar, bioabbaubar und bioabsorbierbar besteht.

5. Wundverband gemäß Anspruch 1, wobei der Wundverband aus einem Material gebildet ist, das aus der Gruppe ausgewählt ist, die aus einem Pulver, einem Film, einem Gewebe, einem Schaum, Garnen, Fasern, einer Beschichtung, einer Lösung, einem Gel oder einer Kombination davon besteht.

6. Wundverband gemäß Anspruch 1, wobei der Wundverband in der Form eines Hämostatikums oder einer Bandage vorliegt.

7. Wundverband gemäß Anspruch 1, der ferner einen Wirkstoff beinhaltet, wobei der Wirkstoff aus der Gruppe ausgewählt ist, die aus einem Antibiotikum, einem Antimikrobiotikum, einem Analgetikum, einem Gerinnungsmittel, einem Steroid, einem Wundheilungsmittel, einem Kühlungsmittel und einem Mittel elektrischen Potentials besteht.

8. Ein Verfahren zum Herstellen eines Wundverbands wie in einem der Ansprüche 1 bis 7 definiert, das Folgendes beinhaltet:
Behandeln von Cellulose mit einer Alkalilösung;
Mischen der Alkalilösung mit einer Lösung aus Chloressigsäure (CAA), um eine Lösung aus gesättigtem Natriumchloracetat (NaCAA) zu bilden, wobei Ionenkristalle in der NaCAA-Lösung gebildet werden; und
Behandeln der Cellulose mit der NaCAA-Lösung, um Carboxymethylcellulose (CMC) mit eingebetteten lonenkristallen zu bilden, wobei ein Gewichtsprozent von lonenkristallen in dem absorbierbaren Polyelektrolytmaterial mindestens etwa 5 Gew.-% eines Gesamtgewichts beträgt.

9. Verfahren zum Herstellen eines Wundverbands wie in einem der Ansprüche 1 bis 7 definiert, das das Assoziieren eines absorbierbaren Polyelektrolytmaterials mit lonenkristallen beinhaltet, wobei ein Gewichtsprozent von Ionenkristallen, das mit dem absorbierbaren Polyelektrolytmaterial assoziiert ist, mindestens etwa 5 Gew.-% eines Gesamtgewichts beträgt.

10. Verfahren gemäß Anspruch 9, wobei die lonensalzkristalle im Wesentlichen mit dem absorbierbaren Polyelektrolytmaterial assoziiert sind.

11. Verfahren gemäß Anspruch 9, wobei das Gewichtsprozent von lonenkristallen etwa 20 bis 70 Gew.-% des Gesamtgewichts beträgt.

12. Verfahren gemäß Anspruch 9, wobei das Polyelektrolytmaterial aus der Gruppe ausgewählt ist, die aus bioresorbierbar, bioabbaubar und bioabsorbierbar besteht.

13. Verfahren gemäß Anspruch 9, wobei der Wundverband aus einem Material gebildet ist, das aus der Gruppe ausgewählt ist, die aus einem Pulver, einem Film, einem Schaum, einem Gewebe, Garnen, Fasern, einer Beschichtung, einer Lösung, einem Gel oder einer Kombination davon besteht.

14. Verfahren gemäß Anspruch 9, wobei der Wundverband in der Form einer Arterienklemme oder einer Bandage ist.

15. Verfahren gemäß Anspruch 9, wobei der Wundverband ferner einen Wirkstoff beinhaltet, wobei der Wirkstoff aus der Gruppe ausgewählt ist, die aus einem Antibiotikum, einem Antimikrobiotikum, einem Analgetikum, einem Gerinnungsmittel, einem Steroid, einem Wundheilungsmittel, einem Kühlungsmittel und einem Mittel elektrischen Potentials besteht.

## Revendications

1. Un pansement pour plaie comprenant un matériau polyélectrolytique absorbable et des cristaux ioniques, dans lequel un pourcentage en poids de cristaux ioniques dans le matériau polyélectrolytique absorbable est d'au moins environ 5 % p/p d'un poids total.

2. Le pansement pour plaie de la revendication 1, dans lequel les cristaux ioniques sont intégrés dans le matériau polyélectrolytique absorbable.

3. Le pansement pour plaie de la revendication 1, dans lequel le pourcentage en poids de cristaux ioniques est d'environ 20 à 70 % p/p du poids total.

4. Le pansement pour plaie de la revendication 1, dans lequel le matériau polyélectrolytique est sélectionné dans le groupe constitué de matériau polyélectrolytique biorésorbable, biodégradable et bioabsorbable.

5. Le pansement pour plaie de la revendication 1, le pansement pour plaie étant formé à partir d'un matériau sélectionné dans le groupe constitué d'une poudre, d'un film, d'un tissu, d'une mousse, de fils, de fibres, d'un revêtement, d'une solution, d'un gel ou d'une combinaison de ceux-ci.

6. Le pansement pour plaie de la revendication 1, le pansement pour plaie étant sous la forme d'un hémostatique ou bandage.

7. Le pansement pour plaie de la revendication 1, comprenant en outre une substance active, la substance active étant sélectionnée dans le groupe constitué d'un antibiotique, d'un antimicrobien, d'un analgésique, d'un agent coagulant, d'un stéroïde, d'un agent de cicatrisation des plaies, d'un agent refroidissant et d'un agent de potentiel électrique.

8. Un procédé de fabrication d'un pansement pour plaie tel que défini dans n'importe laquelle des revendications 1 à 7, comprenant :
le traitement de cellulose avec une solution alcaline ;
le mélange de la solution alcaline avec une solution d'acide chloroacétique (CAA) pour former une solution de chloroacétate de sodium (NaCAA) saturée, dans lequel des cristaux ioniques sont formés dans la solution de NaCAA ; et
le traitement de la cellulose avec la solution de NaCAA pour former de la carboxyméthylcellulose (CMC) ayant des cristaux ioniques intégrés, dans lequel un pourcentage en poids de cristaux ioniques dans le matériau polyélectrolytique absorbable est d'au moins environ 5 % p/p d'un poids total.

9. Un procédé de fabrication d'un pansement pour plaie tel que défini dans n'importe laquelle des revendications 1 à 7, comprenant l'association d'un matériau polyélectrolytique absorbable avec des cristaux ioniques, dans lequel un pourcentage en poids de cristaux ioniques associés avec le matériau polyélectrolytique absorbable est d'au moins environ 5 % p/p d'un poids total.

10. Le procédé de la revendication 9, dans lequel les cristaux de sel ioniques sont substantiellement associés avec le matériau polyélectrolytique absorbable.

11. Le procédé de la revendication 9, dans lequel le pourcentage en poids de cristaux ioniques est d'environ 20 à 70 % p/p du poids total.

12. Le procédé de la revendication 9, dans lequel le matériau polyélectrolytique est sélectionné dans le groupe constitué de matériau polyélectrolytique biorésorbable, biodégradable et bioabsorbable.

13. Le procédé de la revendication 9, dans lequel le pansement pour plaie est formé à partir d'un matériau sélectionné dans le groupe constitué d'une poudre, d'un film, d'une mousse, d'un tissu, de fils, de fibres, d'un revêtement, d'une solution, d'un gel ou d'une combinaison de ceux-ci.

14. Le procédé de la revendication 9, dans lequel le pansement pour plaie est sous la forme d'un hémostatique ou bandage.

15. Le procédé de la revendication 9, dans lequel le pansement pour plaie comprend en outre une substance active, la substance active étant sélectionnée dans le groupe constitué d'un antibiotique, d'un antimicrobien, d'un analgésique, d'un agent coagulant, d'un stéroïde, d'un agent de cicatrisation des plaies, d'un agent refroidissant et d'un agent de potentiel électrique.
